# EUROPEAN PATENT APPLICATION

(11) **EP 2 787 068 A1**
(43) Date of publication of application: **08.10.2014**
(21) Application number: 12853911.1
(22) Date of filing: 27.11.2012
(51) Int. Cl.: C12M 1/00, C12Q 1/68, C12N 15/09

(54) **BASE FOR USE IN AMPLIFICATION OF NUCLEIC ACID, AND NUCLEIC ACID AMPLIFICATION METHOD**

(30) Priority: 01.12.2011 JP 2011263730
(71) Applicant: Mitsubishi Rayon Co., Ltd., Tokyo 100-8253 (JP)
(72) Inventor: OOSHIMA Hiroyuki, Yokohama-shi Kanagawa 230-0053 (JP)
(74) Representative: HOFFMANN EITLE
(86) International application number: PCT/JP2012/080548
(87) International publication number: WO 2013/080941

(57) **Abstract**

The present invention is to provide a base for use in the amplification of a nucleic acid, which makes it possible to accurately and effectively amplify and detect a plurality of target nucleic acid sequences that are subjects for detection. A base for use in the amplification of a nucleic acid according to the present invention is a base in which a hydrophilic gel is held and which has arrayed plural zones, and the zones contain different kinds of primer or the like for amplifying nucleic acids.

## Description

### TECHNICAL FIELD

The present invention relates to a base for use in the amplification of a nucleic acid and a manufacturing method thereof, and a method for amplifying a nucleic acid using the base, and the like.

### BACKGROUND ART

Nucleic acid sequence analysis of genomes of various organisms including human and the like are energetically conducted, and recently gene analysis technology of a large scale sequence, gene screening and the like were rapidly upgraded. In addition, many gene markers associated with a disease or pharmacology have been identified, and practical application of new diagnosis and inspection technique using these has started.

In these gene analyses, diagnosis and inspection, a target nucleic acid sequence that is a subject for the analysis is often amplified, and as the amplification method, PCR (Polymerase Chain Reaction) method, LAMP method (Loop-Mediated Isothermal Amplification) and the like are generally used.

In order to apply these methods for the amplification of a nucleic acid to a large scale sequence, gene screening and the like, suggested are methods and devices for amplifying unspecific sequences independently and comprehensively. In addition, for optimization of the inspection and the diagnosis and the like, suggested are methods and devices for amplifying plural kinds of specific sequences independently.

As an example of the former, a method has been invented in which PCR is implemented inside of a well formed by a capillary penetrated through two sides of a microplate (Patent Document 1). According to the present method, a microplate on which many capillary-shaped wells are formed, is dipped in the PCR solution, and the reaction is conducted such that one molecule of a target nucleic acid is contained in each well, whereby to amplify one target nucleic acid in each well independently.

In addition, a method not using a microplate has been also suggested (Patent Document 2). This method is characterized that many microdroplet gels containing a PCR solution, in which one molecule of a target nucleic acid is contained, are prepared as an emulsion in a hydrophobic solvent such as oil, and are subjected to PCR amplification reaction at this state.

On the other hand, examples of the latter include a multiplex PCR method. In an ordinary PCR method, amplification of a target base sequence is conducted using one kind of primer set for one reaction solution. On the contrary, in a multiplex PCR method, amplifications of plural kinds of target base sequences are conducted using a plurality of primer sets for different amplification subjects in one reaction vessel.

Conversely to this, a method has been also suggested in which plural kinds of primer pairs are placed in independent fine spaces respectively, and PCR is conducted individually in the fine space (Patent Document 3). According to the present method, a basal plate is prepared on which many capillaries are arranged, and plural kinds of primer pairs are dropped and dried, and a PCR solution without primer pairs is contacted with the basal plate and sucked, whereby to redissolve the primer pairs. Therefore it is possible to conduct PCR using different kinds of primers independently in each capillary.

Furthermore, a method has been also invented in which plural kinds of primer pairs are immobilized on a basal plate at their ends, and arrayed, and then amplifications of many target nucleic acid sequences are conducted at one time (Non-Patent Document 1). According to this method, a primer set in which any common adapter-sequence is added to the 5' end side of the primer specific to the target sequence, is immobilized on a basal plate, and further the common adapter-sequence is added as a primer to the liquid phase. Thus, it is possible to amplify and detect those originally difficult to be amplified only with the primers immobilized on a basal plate.

### CITATION LIST

### PATENT DOCUMENTS

Patent Document 1: Japanese Patent Application Laid-Open (JP-A) No. 2002-335960
Patent Document 2: JP No. 2008-245612 A
Patent Document 3: JP No. 2008-154490 A

### NON-PATENT DOCUMENTS

Non-Patent Document 1: Nucleic Acids Research, 2005, Vol. 33, No. 2 e11

### DISCLOSURE OF THE INVENTION

### PROBLEM TO BE SOLVED BY THE INVENTION

However, with these conventional technologies, it is difficult to amplify plural kinds of target sequences in high-throughput without mixing.

For example, with the technology described in Patent Document 1, it is not possible to use plural kinds of primers in one reaction solution, and thus it is difficult to amplify plural kinds of specific sequences. In addition, a capillary having sucked the PCR solution is used, but the PCR solution is immediately dried in the capillary having originally very small quantity of the volume, and thus it is not considered that the reproducibility of the results is high.

With the technology described in Patent Document 2, it is not possible to use a plurality of primer pairs in one reaction solution (in one microdroplet gel) similarly to the technology described in Patent Document 1 although the number of the fragments that can be amplified is very large. In addition, in detection after the amplification reaction, it is necessary to carefully handle the microdroplet gels in the emulsion, and furthermore, it is necessary to identify the kind of the amplification product contained in various microdroplet gels by sequencing additionally, since the kind of the amplification product contained in each of the microdroplet gels is not clear, and thus advanced technology or expensive apparatus is necessary, and practical application is difficult.

With the technology described in Patent Document 3, it is possible to individually implement PCR using plural kinds of primer pairs, but the primer itself is simply dried and adhered in each of the ereas, and thus the open part of the capillary is connected through a water-based solution at the time of contact with the PCR solution, dissolved primer pairs are mixed, and unintended amplification products are likely to be generated. In addition, it is necessary to collect the PCR solution from the capillary at the time of detecting the amplification products. In an experiment system not requiring the collecting, the operations are complicated such that the PCR solution is not leaked from the capillary at the time of the detection, and a labor not to be dried is necessary.

With the technology described in Non-Patent Document 1, the amplification efficiency is improved in comparison to PCR using a carrier on which the primers are immobilized. However, since the reaction at the early stage of PCR is performed only in the solid phase, the amplification efficiency is low and the reaction requires lots of time in comparison to PCR in the aqueous phase. In addition, common primers are used in the liquid phase, and thus many common primers are used in amplification of target nucleic acid sequences originally existing abundantly in the sample, and target nucleic acid sequences originally existing in a small amount may be not detected. In other words, depending on the amount ratio, target nucleic acid sequences not being amplified may exist.

Accordingly, the object of the present invention is to provide a base for use in the amplification of a nucleic acid, which makes it possible to accurately and effectively amplify and detect a plurality of target nucleic acid sequences that are subjects for detection, and a method for amplifying a nucleic acid using the base, and the like.

### MEANS FOR SOLVING PROBLEM

The inventors of the present invention conducted thorough investigations so as to solve such problems described above, and as a result, the inventors found that by using a base having arrayed plural zones, which hold different kinds of primer pairs for use in the amplification of nucleic acids,, a polymerase and a buffer component through a hydrophilic gel that dissolves by heating, it is possible to accurately and effectively amplify and detect a plurality of target nucleic acid sequences that are subjects for detection, thus completing the present invention.

In other words, the present invention is described below.
(I) A base for use in the amplification of a nucleic acid in which a hydrophilic gel that can be dissolved by heating is held, and which has arrayed plural zones, wherein the zone contains different kinds of primer pairs for amplifying nucleic acids, a polymerase and a buffer component.

In the base described in (I) above, the concentration of the primer in each of the zones may be, for example, 1 to 1000 fmol/µL.

In addition, the buffer component may contain at least one kind selected from the group consisting of, for example, KCl, Tris-HCl, MgCl₂, gelatin and Triton X-100.

In addition, the hydrophilic gel (a hydrophilic gel that can be dissolved by heating) may be a gel prepared using at least one kind selected from the group consisting of agarose, alginic acid, dextran, vinyl alcohol and ethylene glycol as a monomer component, and is particularly preferably an agarose gel. Herein, the concentration of the hydrophilic gel may be, for example, 0.5 to 2 mass%.

The size of each of the zones in the base described in (I) above may be set such that the maximum width of the shape of the zone is, for example, 10 µm to 1000 µm.

(II) A method for producing the base for use in the amplification of a nucleic acid described in (I) above, which comprises:
   (1) a step wherein a plurality of hollow fibers are three-dimensionally arranged so that the fiber axial directions of the hollow fibers become the same, and wherein the arrangement is fixed with a resin to produce a hollow fiber bundle;
   (2) a step wherein a plurality of types of gel precursor solutions containing the primers are introduced into the respective hollow portions of the hollow fibers of the hollow fiber bundle that has been heated in advance;
   (3) a step wherein the gel precursor solutions introduced into the hollow portions are reacted to hold a gel-like product comprising the primers in the hollow portions of the hollow fibers; and
   (4) a step wherein the hollow fiber bundle is sliced in the direction crossing the longitudinal direction of the fibers into thin sections in a hydrophobic liquid.
(III) A kit for use in the amplification of a nucleic acid, which includes the base for use in the amplification of a nucleic acid described in (I) above, and a base for use in template supply.
(IV) A method for amplifying a nucleic acid, which includes a process of supplying a template to each of the zones of the base for use in the amplification of a nucleic acid described in (I) above, and a process of heating the base after the supply.

### EFFECT OF THE INVENTION

According to the invention, it is possible to implement the amplification reaction of a nucleic acid using plural kinds of primer pairs, without mixing primer pairs for different target nucleic acid sequences with each other.

In addition, since the primers are not immobilized onto the solid phase, the primers are liberated when the gel is liquefied by heating, and it is possible to implement the amplification reaction at the equivalent speed to the amplification reaction of a nucleic acid in the liquid phase. Due to the water retention potential of the gel, the drying hardly goes on despite the small volume, and the stability of PCR increases.

Furthermore, since a common primer for the target nucleic acid sequence is not used, the amount ratio of original target nucleic acids does not have influence on the results of the amplification, without scramble for the primer between the target nucleic acid sequences that are subject for the amplification reaction.

### BRIEF DESCRIPTION OF DRAWINGS

Fig. 1 is an example of a schematic diagram of a base for use in the amplification of a nucleic acid;
Fig. 2 is a cross-sectional view that illustrates an example of the base for use in the amplification of a nucleic acid;
Fig. 3 is a cross-sectional view that exemplifies the state in which both ends of the base for use in the amplification of a nucleic acid are sealed with sheets;
Fig. 4 is a drawing that exemplifies an embodiment at the time of the amplification reaction of a nucleic acid using the base for use in the amplification of a nucleic acid;
Fig. 5 is a drawing that illustrates signal intensity of SybrGreen I detected by PCR using the base for use in the amplification of a nucleic acid; and
Fig. 6 is a drawing that illustrates the experimental results of Example 2 of the present application, specifically the ratio of an agarose gel supported on each of the zones of the base for use in the amplification reaction of a nucleic acid obtained by the slicing (the residual ratio).

### BEST MODE(S) FOR CARRYING OUT THE INVENTION

Hereinafter, the present invention will be explained in detail. The scope of the present invention is not limited to the explanation, and may be implemented with suitable change even beyond the examples described below within a scope where the intention of the present invention is not impaired.

Further, the specification of Japanese patent application No. 2011-263730 (filed on December 1, 2011) and the like, on which the claiming for the priority of the present application is based, are incorporated herein in its entirety. In addition, all of the publications, for example, the prior art documents, and the open publications, the patent publications and the other patent documents cited in the present specification are incorporated herein by reference.

### 1. Base having arrayed plural zones for use in amplification of nucleic acid

A material (raw material) for the base for use in the amplification of a nucleic acid (hereinafter, it may be referred to as "base") used in the present invention is, for example, preferably those capable of maintaining the shape even under a temperature of 100°C or higher, more preferably those capable of maintaining the shape even under a temperature of 110°C or higher since the base for use in the amplification of a nucleic acid is used as a reaction vessel for which the heat treatment is conducted. Examples of such material include, for example, glass, silicon, resin and the like. As the resin, an epoxy resin, a polypropylene resin, a polystyrene resin, a polyurethane resin and the like may be used.

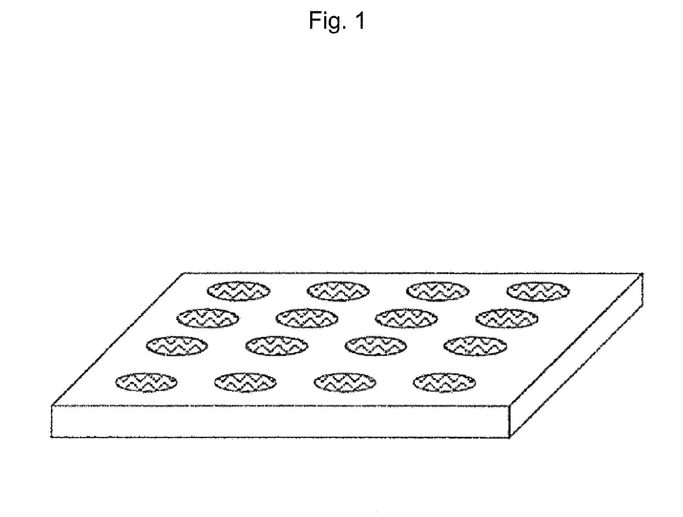

The shape of the base is not particularly limited. Examples of the shape of the base include, for example, a cube, a cuboid (for example, planar shape as exemplified in Fig. 1), a polygonal prism, a triangular prism, a column, an elliptic cylinder and the like. The shape of the base is preferably a cube or a cuboid from the viewpoint of the handling property and the compatibility with a commercially available instrument at the time of the reaction or the detection.

The size of the base is also not particularly limited. If a heating instrument such as a commercially available thermal cycler is used, the size of the base is preferably, for example, set such that 24 pieces or more of the base can be installed in the instrument, and more preferably 96 pieces or more of the base can be installed in terms of the high throughput. Accordingly, if the base has a planar structure of a square or a rectangle, the base is preferably rectangular having 4 to 14 mm for one side (for the longer side in the case of the rectangle), and more preferably rectangular having 6 to 12 mm for one side.

The thickness of the base is also not particularly limited, and is preferably those usable in a heat instrument such as a commercially available thermal cycler. For example, the thickness of the base is preferably 1 µm to 5000 µm, more preferably 10 µm to 2000 µm, further preferably 100 µm to 500 µm, and is also preferably 100 µm to 250 µm if the case may be.

The base of the present invention has a plurality of the zones. The shape of the zone is not particularly limited, and examples of the shape include a cube, a cuboid, a column, an elliptic cylinder, a triangular pyramid, a quadrangular pyramid, a hemisphere, and the like. The shape of the zone is preferably a cube, a cuboid, a column or the like among these, from the viewpoint of good workability, easy manufacture and the like.

The size of the zone is also not limited, and may be suitably selected depending on, for example, the size of the base, the number of the zones established in the base, and the like. For example, the shape of the zone is set such that the maximum width of the shape (specifically, the internal diameter in the case of a column, the length of one side in the case of a cube, the length of the longer side in the case of a cuboid) is preferably about 10 µm to 1000 µm, more preferably about 90 µm to 500 µm, and further preferably about 150 µm to 180 µm, respectively.

The depth of the zone is also not particularly limited, and may be suitably selected depending on the thickness of the base and the like. For example, the depth of the zone is preferably 1 µm to 5000 µm, more preferably 10 µm to 2000 µm, and further preferably 100 µm to 500 µm, and is also preferably 100 µm to 250 µm if the case may be.

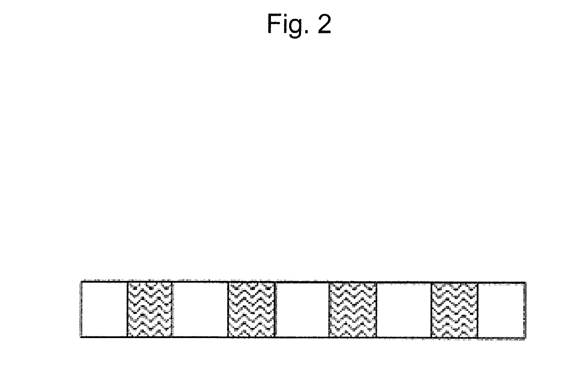

In the base of the present invention, the zones may penetrate the base as exemplified in the cross-sectional view of Fig. 2, or may not penetrate the base. If the zone penetrates the base, the depth of the zone becomes identical to the thickness of the base. On the other hand, if the zone does not penetrate the base, the depth of the zone becomes less than the thickness of the base.

It is preferable to install the zones on the base as many as possible in order to amplify many target nucleic acid sequences at one time with one base. The number of the zones is preferably 50 or more, more preferably 100 or more, and further preferably 200 or more.

In the present invention, a plurality of the zones contained in the base are arrayed. The array is meant to encompass the meaning that, for example, it is clear which kind of primer pairs is contained (placed) in what zone.

The surface of the base may be conducted with various treatments as long as the amplification reaction of a nucleic acid can be implemented. For example, the surface of the base may be performed with water-repelling process by coating a water-repellent resin on the plane of the base, or may be hydrophilized by plasma treatment, or may be performed with sterilization treatment with gamma ray irradiation and the like.

### 2. Hydrophilic gel

The kind of the gel held in the zone of the base of the present invention is not limited as long as the amplification reaction of a nucleic acid can be conducted. A hydrophilic gel is preferably used since an enzyme reaction is necessarily conducted. The hydrophilic gel is preferably those that can be dissolved by heating, and specifically preferably those existing as a gel at approximately room temperature (preferably 25°C or lower, more preferably 30°C or lower, further preferably 35°C or lower) and existing as the liquid phase (solution) at a temperature where the amplification reaction of a nucleic acid is implemented (for example, under heating conditions of preferably 60°C or higher, preferably 50°C or higher, and further preferably 40°C or higher). This is because the reaction effectively proceeds in the solution (in a hydrophilic gel that has been dissolved by heating) at the time of the amplification reaction of a nucleic acid, and formation of the gel easy handling at other times.

Among such hydrophilic gels, the gel is preferably those prepared using agarose, alginic acid, dextran, vinyl alcohol, ethylene glycol and the like as a monomer component. These monomer components may be used alone, or may be used in combination of two or more kinds.

In addition, the gel itself is preferably made of a material not inhibiting the amplification reaction of a nucleic acid. From such viewpoint, the gel is particularly preferably, for example, an agarose gel including Agarose LO3 manufactured by TAKARA BIO INC.

The concentration of the gel used in the present invention is preferably 0.5 to 2 mass%, more preferably 0.7 to 1.8 mass%, and further preferably 0.9 to 1.6 mass%. This is because if the concentration of the gel is 0.5 mass% or more, remarkable decrease of the gel intensity is avoided. 2 mass% or less of the concentration of the gel is set because the gel can be easily re-dissolved when the temperature is increased.

In the base of the present invention, the hydrophilic gel described above is held in each of the zones together with the solution for the amplification reaction of a nucleic acid containing the primers for use in the amplification of a nucleic acid described below and the like. In other words, the solution for the amplification reaction of a nucleic acid is contained in the hydrophilic gel in each of the zones after the holding.

### 3. Primer for amplifying nucleic acid, and the like

Different kinds of primer pairs need to be loaded in each of the zones of the arrayed plural zones in order to amplify plural kinds of target nucleic acid sequences. The primer itself may be a oligonucleotide consisting of DNAs, or may be RNA, PNA, LNA and the like if they have no influence on the specificity and reactivity.

These different kinds of the primer pairs are desirably adjusted in the GC content and the sequence length, and designed, respectively such that the reaction conditions for the amplification reaction of a nucleic acid are equivalent between the primers as long as the specificity necessary for the amplification of the target nucleic acid sequences can be ensured. For example, this is because if the annealing temperatures or the lengths of the amplification products, which are the target sequences, are remarkably different, suitable reaction temperatures or times are different for the respective zones, and it is considered that there is a possibility that simultaneous reaction may become difficult. The design of such primers can be suitably implemented by a person skilled in the art.

One set of the primer pairs may be contained only in one spot of the zone, or may be contained in a plurality of the zones. By loading the same primer pair to a plurality of the zones, it is possible to verify the reproducibility, and to determine the quantity of the target nucleic acid from the numbers of the zones admitted for the amplification and the zones not admitted for the amplification.

The primers may be modified such as amination, biotinylation and fluorescent label as long as the primers can be used in the amplification reaction of a nucleic acid. In addition, two or more kinds of the primer pairs may be contained in one zone depending on the purpose.

The concentration of the primer contained in each of the zones is not particularly limited, and may be suitably selected depending on the kind of the target nucleic acid, the kind of the enzyme used in the amplification reaction, and the like. For example, the concentration of the primer is preferably 1 to 1000 fmol/µL, more preferably 10 to 100 fmol/µL, and further preferably 20 to 50 fmol/µL. By setting the concentration of the primer to 1 fmol/µL or more, it is possible to effectively amplify the sequence of the target nucleic acid. In addition, by setting the concentration of the primer to 1000 fmol/µL or less, it is possible to suppress non-specific amplification or formation of a primer dimer.

Further, in the solution for the amplification reaction of a nucleic acid held in the zone together with the hydrophilic gel, various components in addition to the primers described above, for example, dNTP, a buffer component, DNA polymerase and the like may be contained in advance, and an intercalator specific for a double-stranded nucleic acid such as SYBR Green I, EtBr and PicoGreen may be also contained in advance as the case may be. Glycerol may be also contained in order to increase the water retention of the gel. A template solution may be also introduced in advance unless plural kinds of samples are treated.

Herein, the buffer component is not limited, and examples of the buffer component preferably include a mixture of 50 mM KCl, 10 mM Tris-HCl (pH 8.4 to 9.0, 25°C), 1.5 mM MgCl₂ and 0.01 % gelatin or 0.01 % Triton X-100, and the like.

In addition, the DNA polymerase is not limited, and examples thereof preferably include poll type heat-resisting DNA polymerases such as Taq polymerase and Tth polymerase, or α type heat-resisting polymerases such as Pfu polymerase and KOD polymerase, and the like.

### 4. Manufacture of the base of the present invention

In the base for use in the amplification reaction of a nucleic acid of the present invention, the zones may be formed after manufacturing the base, or the base may be manufactured using hollow tubes or fibers. For example, the base may be manufactured at low cost and in large quantities by the method comprising the step described below.
(1) a step wherein a plurality of hollow fibers are three-dimensionally arranged so that the fiber axial directions of the hollow fibers become the same, and wherein the arrangement is fixed with a resin to produce a hollow fiber bundle;
(2) a step wherein a plurality of types of gel precursor solutions containing the primers are introduced into the respective hollow portions of the hollow fibers of the hollow fiber bundle that has been heated in advance;
(3) a step wherein the gel precursor solutions introduced into the hollow portins are reacted to hold a gel-like product comprising the primers in the hollow portins of the hollow fibers; and
(4) a step wherein the hollow fiber bundle is sliced in the direction crossing the longitudinal direction of the fibers into thin sections in a hydrophobic liquid.

Examples of the fiber that can be used in the present method include synthetic fibers, semisynthetic fibers, regenerated fibers, natural fibers and the like. Typical examples of the synthetic fiber include various fibers such as nylon 6, nylon 66 and aromatic polyamide, polyester-based various fibers such as polyethylene terephthalate, polybutylene terephthalate, polylactic acid, polyglycolic acid, and polycarbonate, acryl-based various fibers such as polyacrylonitrile, polyolefin-based various fibers such as polyethylene and polypropylene, polyvinyl alcohol-based various fibers, polyvinylidene chloride-based various fibers, polychloride vinyl-based fibers, polyurethane-based various fibers, phenol-based fibers, fluorine-based fibers comprising polyvinylidene fluoride, polytetrafluoroethylene and the like, polyalkylene peroxybenzoate-based various fibers and the like. Examples of the semisynthetic fiber include cellulose-based various regenerated fibers using diacetate, triacetate, chitin, chitosan and the like as a raw material (rayon, cupra, polynosic and the like), and the like. Typical examples of the natural fiber include vegetable fibers such as cotton and flax, animal fibers such as wool and silk, mineral fibers such as asbestos, and the like.

As the shape of the fiber, a known hollow fiber may be used. The diameter of the fiber is preferably 1 mm or less, and tens of micron or more. In addition, the inner wall of the zone thus prepared may be covered with a substance having an inhibitory action against protein adsorption such as BSA to prevent adsorption of polymerase and the like. The fiber may be sterilized with radiation treatment and the like, and may be hydrophilized with plasma treatment and the like.

The sentence that the organism-related substance is held through the gel refers that if the fiber is, for example, a hollow fiber, the gel is held in the hollow part using chemical or physical interaction, and the organism-related substance is held in the held gel using chemical or physical interaction. The gel that can be used in the present method is not particularly limited, and, for example, agarose, alginic acid, dextran, polyvinyl alcohol, polyethylene glycol and the like can be used.

Next, the hollow fiber bundle in which a plurality of the fibers are embedded, will be explained.

In arranging a plurality of the fibers orderly such that each axis of the fiber is nearly the same to each other, the fibers are arranged regularly using, for example, the arrangement jig described in WO 00/53736 A. The number of the fibers constituting the arrangement may be 10 to 1,000,000 per 1 cm². The number of the fibers may be suitably selected depending on the number of the zones of the base of the present invention.

An embedding agent is injected homogeneously between the arranged fibers and cured in order to immobilize the arranged fibers. Examples of the kind of the embedding agent include embedding agents consisting of crosslinking prepolymers, embedding agents consisting of polymerization oligomers and catalysts, and thermoplastic polymers and the like.

Examples of the embedding agent consisting of crosslinking prepolymers include polyurethane resins. The polyurethane resin may be prepared by adding a curing agent to polyisocyanate of the crosslinking prepolymer that is the main agent. Examples of the curing agent include alcohols, ketones, amides, esters, castor oil-based polyols and the like. Among these, those having a boiling point of 60°C or higher are particularly preferable, and specific examples thereof may include glycerol, polyethylene glycol, methylethyl ketone, acetamide, N,N-dimethyl formamide, ethyl acetate and the like. In addition, the polyurethane resin, an epoxy resin, a silicon resin and the like may be used besides the polyurethane resin.

Examples of the embedding agent consisting of polymerizable oligomers and catalysts include acryl-based syrups containing (meth)acrylic acid ester [A] and (meth)acrylic-based polymer or copolymer [B] soluble in the component [A].

Specific examples of the component [A] described above include, for example, acrylic acid esters such as methyl acrylate, ethyl acrylate, n-butyl acrylate, acrylate t-butyl and 2-ethylhexyl acrylate; and methacrylic acid esters such as methyl methacrylate, ethyl methacrylate, n-butyl methacrylate, i-butyl methacrylate, t-butyl methacrylate, lauryl methacrylate, 2-ethylhexyl methacrylate, tridecyl methacrylate, stearyl methacrylate, cyclohexyl methacrylate, benzyl methacrylate, 2-hydroxyethyl methacrylate, 2-hydroxypropyl methacrylate, dimethylaminoethyl methacrylate, diethylaminoethyl methacrylate, glycidyl methacrylate, tetrahydrofurfuryl methacrylate and allyl methacrylate. These may be used in one kind or in a mixture of two or more kinds.

If the glass transition temperature (temperature where the hard state changes to the soft state) of the resin obtained by curing any monomer described above alone (one kind only) as the component [A] is low, the obtained cured product tends to be soft, and if the glass transition temperature is high, the obtained cured product tends to be hard. Accordingly, the component [A] is preferably used by suitable selection based on the glass transition temperature of this component [A] as a homopolymer to express desired hardness of the embedding agent.

The (meth)acrylic-based polymer used as the component [B] described above needs to be soluble in the component [A]. Further, the "soluble" includes the dispersion state as well. Specific examples of the component [B] include, for example, homopolymers or copolymers of the monomers selected from methyl methacrylate, ethyl methacrylate, n-butyl methacrylate, i-butyl methacrylate, t-butyl methacrylate, lauryl methacrylate, 2-ethylhexyl methacrylate, methyl acrylate, ethyl acrylate, n-butyl acrylate, t-butyl acrylate, 2-ethylhexyl acrylate and the like. These may be used in one kind or in a mixture of two or more kinds. The use ratio of the component [A] and the component [B] is such that the component [A] is in a range of 40 to 80 mass parts and the component [B] is in a range of 20 to 60 mass parts, and preferably the component [A] is in a range of 40 to 70 mass parts and the component [B] is in a range of 30 to 60 mass parts when the acryl-based syrup consisting of the component [A] and the component [B] is 100 mass parts.

In addition, it is preferable that a urethane oligomer, a urethane polymer or other gum components be suitably added to the acryl-based syrup consisting of the component [A] and the component [B] in conducting the slicing. As a polymerization initiator for the acryl-based syrup, azo-based, peroxide-based and redox-based initiators and the like, which can be dissolved in the solvent to be used, may be used. Examples thereof include 2,2'-azobisisobutyronitrile, 2,2'-azobis(2-methylbutyronitrile) isobutyronitrile, benzoyl peroxide, or benzoyl peroxide-dimethyl aniline-based initiators and the like.

Examples of the embedding agent that is the thermoplastic polymer include polyethylene, polypropylene, polybutylene, ABS resins, methacrylic resins, polyvinyl chloride, polyamide, polycarbonate, polyacetal, PBT resins, polyphenylene sulfide, polyarylate polysulfone, polyether sulfone, polyether ether ketone, polymethylpentene, polyether imide and the like.

When the embedded product embedded by the embedding agent is used as the base for use in the amplification reaction of a nucleic acid, it may be demanded to have low fluorescent intensity in detection of an organism-related substance. In such a case, carbon black, a quencher and the like are preferably added to the embedding agent. In addition, the embedding agent is preferably selected in consideration of the adhesion between the fibers and the embedding agent depending on the kind of the fiber to be used. For example, an embedding agent consisting of the acrylate-based syrup is preferable for polymethyl methacrylate hollow fibers.

The curing temperature of the embedding agent is preferably not higher than the glass transition temperature of the fiber material. The curing temperature of the embedding agent is further preferably not higher than 100°C. Such embedded product in which a plurality of fibers are embedded is sliced in a direction intersecting with the fibers with a hardness of 70 to 95 (measured by JIS K 7215), whereby to obtain the slice of the fiber-arranged body, wherein the surface of the slice is smooth, and the organism-related substance is held without shedding of the fibers. The hardness of the embedding agent is measured by JIS K 7215, and refers to hardness measured after five seconds from the adhesion of the pressing face to the embedded product.

When the gel is held on the base having the zone in which the hollow fibers are used, a gel precursor solution may be filled into the hollow fiber bundle that has been heated in advance, and then gelated in the hollow fiber bundle. Herein, the gel precursor solution refers to a solution that is not gelated containing the primer pairs. In addition, the temperature of the hollow fiber bundle heated in advance may be suitably set depending on the kind of the gel precursor solution to be filled, and the like, and is not limited, but preferably, for example, 50 to 90°C, more preferably 60 to 80°C, and further preferably 70 to 75°C. By heating the hollow fiber bundle in advance, it is possible to obtain an effect of extremely smoothly introducing the gel precursor solution into the hollow fiber bundle, and the like. Then, the hollow fiber bundle holding the gel-like product is sliced in a direction intersecting with the longitudinal direction of the fibers (specifically, sliced in the vertical direction with respect to the orientation direction of the through-hole of the hollow fiber bundle) in the hydrophobic liquid, whereby to obtain the base for use in the amplification reaction of a nucleic acid in which the gel is held. Herein, the hydrophobic liquid is not limited, and is preferably, for example, mineral oil, liquid paraffin and the like. By conducting the slicing in the hydrophobic liquid, a base of high quality is obtained, in which the gel-like product is sufficiently held in the hollow fiber even after the slicing.

A method for filling the gel precursor solution into the hollow fiber bundle may be conducted by, for example, sucking the solution into a syringe having a fine needle and introducing the needle into the hollow part of each hollow fiber.

In addition, when one of the end parts of the hollow fiber bundle is not immobilized in a case where a hollow fiber bundle manufactured by binding hollow fibers with a resin is used, the gel precursor solution may be introduced into the hollow fiber by the following method. Firstly, the hollow part of immobilized end part of the hollow fiber bundle is sealed, and the hollow part of the other non-immobilized end part is opened. Next, the gel precursor solution containing the primer pairs is prepared, and the gel precursor solution and the hollow fiber bundle are installed in a desiccator, and subsequently the non-immobilized end part of the hollow fiber of the hollow fiber bundle is soaked in this solution, and the inside of the desiccator is rendered to be in a decompression state, and then returned to ordinary pressure, whereby to introduce this solution into the hollow part of the hollow fiber from the end part of the hollow fiber soaked in the solution.

The gel precursor solution introduced to the hollow part of the hollow fiber is gelated, whereby to hold the primer pairs in the hollow part of the hollow fiber. The gelation conditions are not particularly limited, and may be suitably selected depending on the kind of the gel precursor used and the like. For example, if the gel precursor is agarose, the gel precursor may be gelated by cooling the gel precursor solution, which is a liquid by heating in advance, to a temperature not higher than the gelation temperature.

Next, slicing of the embedded product in which the fibers are arranged, will be described.

The embedded product is cut in a direction intersecting with the fiber axis of the embedded product in the slicing. The cutting angle is not limited, and may be suitably selected depending on the purpose of the experiment and the like. For example, the cutting angle is 45 to 90°, preferably 60 to 90°, and further preferably the orthogonal direction to the fiber axis (90° to the fiber axis).

The cutter edge angle of the cutter used in the cutting is preferably 20° or less, and further preferably 15° or less. As the material for the cutter, for example, carbon steel, SUS, cemented carbide and the like can be exemplified. In addition, a commercially available microtome may be used as the apparatus in implementing the slicing. The thickness of the slice is as described above.

Thus-prepared slice can be used as the base for use in the amplification reaction of a nucleic acid for amplifying many target nucleic acid sequences.

### 5. Use of the base of the present invention

A method for amplifying and detecting a nucleic acid using the base for use in the amplification reaction of a nucleic acid of the present invention may be a PCR method in which heat cycle is used, or LAMP method of isothermal reaction if the method is a method using primers. Setting of the conditions for the amplification reaction of a nucleic acid, design of the primers, and the like in these techniques may be suitably implemented by a person skilled in the art. However, the reaction temperature needs to be not lower than the melting point of the gel in the base for use in the amplification reaction of a nucleic acid.

A method for using the base for use in the amplification of a nucleic acid in the amplification reaction of a nucleic acid may be the method shown below.

### Supply of template

Firstly, nucleic acids (hereinafter, referred to as "template") containing target base sequences, which is a sample are supplied into each of the zones of the base of the present invention before initiation of the reaction. The template may be supplied as a solid such as a powder, or may be supplied as a solution.

Any template may be used if the template is a nucleic acid that is a template used in an ordinary amplification reaction of a nucleic acid. A template derived from a natural product is ordinarily used in order to investigate the contained nucleic acid, but nucleic acids purified therefrom may be used, or a liquid of crushed cells or tissues or a supernatant thereof may be used depending on the use. In addition, artificially synthesized nucleic acid may be also used as the template. A reverse transcription product from mRNA may be also used as the template.

The template may be supplied as a solution to each of the zones using a dispensing appliance such as a pipette. By this, it is possible to prevent liberation and mixing of the primers dispensed into each of the zones. The template may be also supplied to each of the zones of the base using an apparatus such as an arrayer and a manipulator.

On the other hand, the template may be also supplied at one time to each of the zones by dropping the template solution on a material for use in template supply and drying the material whereby to coat the material for use in template supply, and attaching the material for use in template supply to the surface of the base. In this case, the shape of the template may be a plane shape, or may be a sheet shape or film shape.

If the material for use in template supply is subjected to the reaction as attached to the base of the present invention, the material for use in template supply needs to have heat-resistance so as to be used in the amplification reaction of a nucleic acid, and is desirably a material not inhibiting the detection method applied in order to directly detect the amplification product. In addition, the material for use in template supply is desirably crimpable on the base surface in addition to the gel for the simplicity of the use thereof.

Examples of such material for use in template supply include films made of polyolefin and the like. In addition, films such as those used in sealing wells in real time PCR and the like may be also used.

A method for coating the material for use in template supply to the template may use a tool for handling liquid such as a pipette, or may be a method in which the sample solution is adsorbed with a cotton swab, a pencil, a paper point and the like, and daubed on a film. The template solution dropped on the film may be coated with rotation in the central part in order to be coated homogeneously.

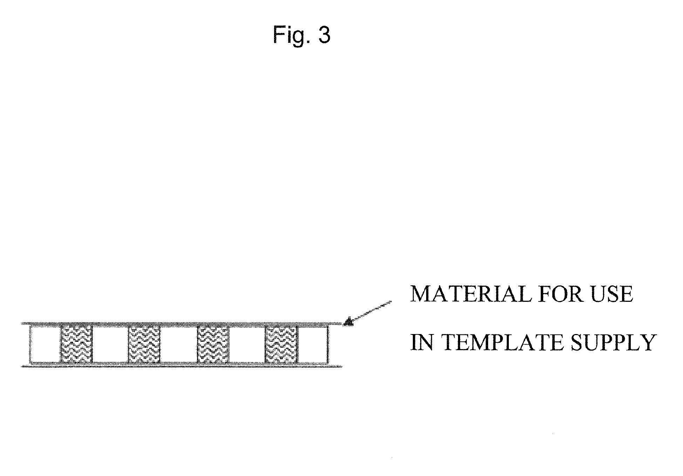

If the zone does not penetrate the base when the template solution is supplied, the material for use in template supply containing the template can be brought into contact with the side that is in contact with the zone. In addition, if the zone penetrates the base as exemplified in the cross-sectional view of Fig. 2, the material containing the template for use in template supply can be brought into contact with both sides of the base, or the material containing the template for use in template supply can be brought into contact with either one side, and the material not containing the template can be brought into contact with the other side (Fig. 3).

The material for use in template supply may be implemented with various surface treatments as necessary. For example, by covering (treating) the material for use in template supply with a blocking agent such as salmon sperm DNA, it is possible to prevent adhesion of a nucleic acid that is the template to the surface of the base for use in template supply. The material for use in template supply may be also sterilized with radiation treatment and the like, or hydrophilized with plasma treatment and the like.

The base for use in the amplification reaction of a nucleic acid that has been brought into contact with the template as described above may be inserted into a heat source for one side or both sides of the base whereby to implement the amplification reaction of a nucleic acid. In this case, the ends of the zone need to be sealed so that the gel in the zone or the lysate of the gel is not leaked.

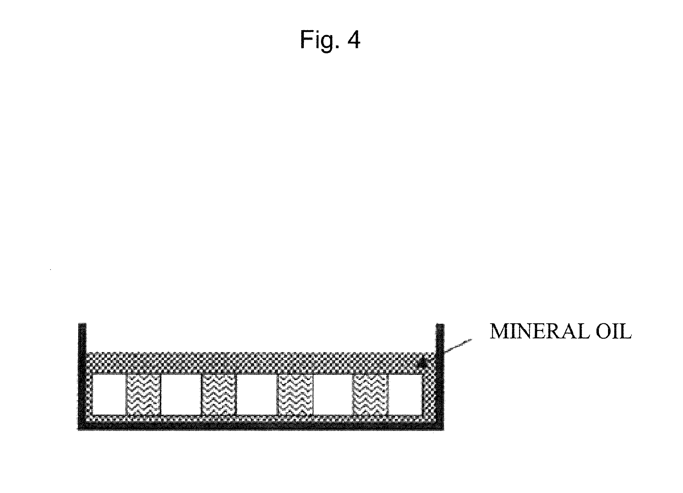

In addition, for example, the amplification reaction of a nucleic acid may be also implemented with the base for use in the amplification reaction of a nucleic acid embedded (dipped) in the hydrophobic liquid so that the content of the gel such as mineral oil is not leaked as exemplified in Fig. 4. Since the mineral oil forms the boundary with the gel solution, the ends of the zone do not need to be sealed. In addition, in this case, the film used for contact with the template may be stripped off and then the base for use in the amplification reaction of a nucleic acid may be subjected to the amplification reaction of a nucleic acid.

The presence or absence of the amplification may be easily checked using a commercially available fluorescent detector, a fluorescent microscope and the like with putting in an intercalator emitting fluorescence specifically for the double strand of the nucleic acid at the time of the reaction.

Furthermore, the gel in the inside may be extracted by centrifuge together with mineral oil after the amplification. The extracted gel may be dissolved, and the amplified nucleic acids in the inside may be acquired. The zones not intended to be acquired may be sealed in advance whereby to acquire the intended gel only.

### Heating of base

The base after the template supply is heated whereby to conduct the amplification reaction of a nucleic acid. The reaction conditions (heating conditions) for amplifying the nucleic acids are not limited, and may be suitably selected depending on enzymes to be used (polymerase and the like), the template and the like. For example, if a PCR method is conducted, the heat cycle may be exemplified by repetition of 30 cycles or so of a heating process of 98°C for 10 seconds for separating the double-stranded nucleic acid, 55°C for 30 seconds for the annealing, and 72°C for 1 minute for the extension reaction of the nucleic acids, and the like. The temperature, the time and the cycle number in each process can be suitably selected by a person skilled in the art. In addition, if a LAMP method is conducted, the heating can be exemplified by, for example, isothermal heating at 50 to 70°C, and the heating time can be suitably selected by a person skilled in the art.

### 6. Kit

The base of the present invention may be used as a kit for use in the amplification of a nucleic acid. The kit preferably comprises the base of the present invention and the base for use in template supply.

Hereinafter, the present invention will be described more specifically with Examples, but the present invention is not limited to these Examples.

### Example 1

### Manufacture of resin block

2 Pieces of porous plates having 0.1 mm of the thickness, in which 228 holes having 0.32 mm of the diameter were arranged in 19 x 12 grid shape at 0.42 mm of the interval in the center of the plate, were passed with hollow fibers made of polycarbonate colored with carbon black (manufactured by Mitsubishi Rayon Co., Ltd., 0.28 mm of the outer diameter, 0.18 mm of the internal diameter and 500 mm of the length) through all of the holes of the porous plates. These 2 pieces of the porous plates passed with the fibers were separated by 50 mm, and a polyurethane resin colored with carbon black (NIPPOLAN 4276 and CORONATE 4403 manufactured by NIPPON POLYURETHANE INDUSTRY, CO. LTD.) were filled between the separated porous plates, and a resin block having 50 mm of the length and having parts not immobilized with a resin at prism-shaped both ends of 7 x 12 mm angle was obtained.

### Preparation of PCR solution

3 kinds of primer pairs for different detection subjects, specifically, total six kinds of primers (A1 (SEQ NO: 1), A2 (SEQ NO: 2), B1 (SEQ NO: 3), B2 (SEQ NO: 4), C1 (SEQ NO: 5) and C2 (SEQ NO: 6) to be described below) were prepared in 10 pmol/µL of the concentration, respectively, and three kinds of solutions for the amplification reaction of nucleic acids according to the primer pairs were prepared using PCR premix containing dNTP, the buffer and DNA polymerase (PremixTaq manufactured by TAKARA BIO INC.), agarose (LO3 manufactured by TAKARA BIO INC.), SYBR Green I (manufactured by Life Technologies), Streptavidin-Cy5 (manufactured by GE Healthcare) and ultrapure water (see Table 1 below).

Primer A1: caggcagacggaagagttcg (SEQ NO: 1)
Primer A2: ttatcacgtccttcttcgcc (SEQ NO: 2)

Primer B1: cagatatttgacataactagggaag (SEQ NO: 3)
Primer B2: ctttcgcttgaccatattattgtcc (SEQ NO: 4)

Primer C1: gaaactatatagtagaacaaacaag (SEQ NO: 5)
Primer C2: atcggctcttactacctaggc (SEQ NO: 6)

**[Table 1]**

| | Concentration | A | B | C | |
|---|---|---|---|---|---|
| Agarose (TaKaRa) | - | 7.5 mg | 7.5 mg | 7.5 mg | mg |
| D.W. | - | 184 | 184 | 184 | ul |
| ExTaq ver2 premix(TaKaRa) | 0.05 U/ul | 250 | 250 | 250 | ul |
| Primer A1 | 10 pmol/ul | 2.5 | | | ul |
| Primer A2 | 10 pmol/ul | 2.5 | | | ul |
| Primer B1 | 10 pmol/ul | | 2.5 | | ul |
| Primer B2 | 10 pmol/ul | | 2.5 | | ul |
| Primer C1 | 10 pmol/ul | | | 2.5 | ul |
| Primer C2 | 10 pmol/ul | | | 2.5 | ul |
| Streptavidin-Cy5(GE healthcare) | 1 ug/ul | 1 | 1 | 1 | ul |
| SYBR Green I(Lifetechnologies) | 1000 times diluted | 50 | 50 | 50 | ul |

### Introduction of solution for amplification reaction of nucleic acid into resin block

The prepared three kinds of the solution for the amplification reaction of a nucleic acid were heated with a heat block at 80°C for 5 minutes, and agarose was dissolved. In the solution for the amplification reaction of a nucleic acid in which the agarose was dissolved, hollow fibers protruded from the end of a resin block heated to 65°C in advance were dipped every 5 pieces, and the gel solution was absorbed from the opposite end of the hollow fiber, whereby to introduce the solution for the amplification reaction of a nucleic acid into the resin block.

The resin block was cooled to room temperature, and the solution for the amplification reaction of a nucleic acid in the inside was gelated.

### Acquisition of base for use in amplification reaction of nucleic acid by slicing

The above-obtained resin block filled with the gel consisting of the solution for the amplification reaction of a nucleic acid containing the three kinds of the primer pairs was cut to 250 µm of the thickness using a microtome, and a base for use in the amplification reaction of a nucleic acid was obtained in which total 228 holes of 12 (height) x 19 (width) were regularly arranged. The both sides of the obtained base for use in the amplification reaction of a nucleic acid were sealed with a cellophane tape for dry spinning.

### Coating of template solution on sheet

DNAs consisting of the target nucleic acid sequence A (SEQ NO: 7) amplifiable only with a combination of the primers A1 and A2 were prepared at a concentration of 1 amol/µL as the template solution.

Target nucleic acid sequence A:

caggcagacggaagagttcgctatagctcagttggttagagcgctacactgataatgtagaggtcggcagttcaactct
gggcgaagaaggacgtgataa (SEQ NO: 7).

10 µL of the template solution was dropped to a sheet for crimping cut to 1 cm angle (Micro Optical Adhesive film manufactured by Life Technologies), and coated on the sheet with the tip of a pipette, and stood and dried at room temperature.

### Contact of base for use in amplification reaction of nucleic acid with sheet finished with template coating

The cellophane tape on one side of the base for use in the amplification reaction of a nucleic acid was peeled off, and a sheet coated with the template solution was crimped on the same side. Then, the cellophane tape on the opposite side was peeled off, and the base for use in the amplification reaction of nucleic acid was dipped into a sample tube containing 50 µL of mineral oil (QIAGEN).

### Detection before implementing PCR

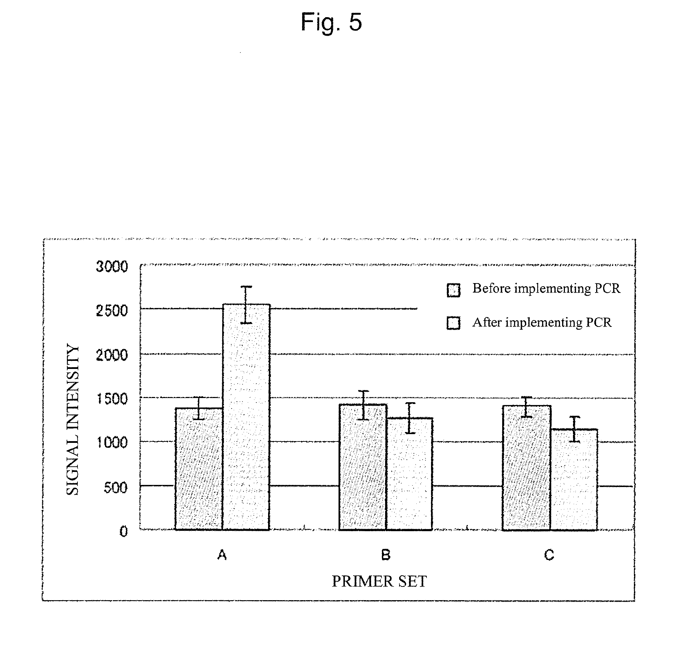

Before implementing PCR, the fluorescence of SYBR Green I intercalated specifically to the double-stranded nucleic acid was detected with a mirror unit (WIB-UMWIB3 manufactured by OLYMPUS CORPORATION) using a fluorescent microscope, and imaged with a CCD camera, and then the intensity of the fluorescence in each hole was digitized with the image treatment software (ImagePro manufactured by Media Cybernetics) (Fig. 5). Since the PCR reaction is not implemented, a double-stranded nucleic acid is not produced, and thus the signal obtained herein may be regarded as the background.

### Implementing of PCR

An Eppendorf tube in which the base for use in the amplification reaction of a nucleic acid was dipped, was set to a thermal cycler, and 30 cycles were repeated, in which the one cycle consists of 98°C for 10 seconds, 55°C for 30 seconds, and 72°C for 1 minute.

### Detection of amplification product

The fluorescence of SYBR Green I intercalated specifically to the double-stranded nucleic acid was detected with a mirror unit (WIB-UMWIB3 manufactured by OLYMPUS CORPORATION) using a fluorescent microscope, and imaged with a CCD camera, and then the intensity of the fluorescence in each hole was digitized with the image treatment software (ImagePro manufactured by Media Cybernetics). Thus, it was found out that significant increase of the signal intensity was found out only for the zone into which the primer pair A was introduced among the three kinds of the primer pairs.

Accordingly, it was found out that specific amplification was performed only with the gel holding the primer pair A among the three kinds of the primer pairs held in the base for use in the amplification of a nucleic acid used (Fig. 5).

### Example 2

The resin block and the solution for the amplification reaction of a nucleic acid were prepared with a method similar to the method described in Example 1. However, with respect to the solution for the amplification reaction of a nucleic acid, the primers were not contained, and single composition of the solution was used in this Example.

### Introduction of solution for amplification reaction of nucleic acid into resin block

The prepared solution for the amplification reaction of a nucleic acid was heated at 80°C for 5 minutes with a heat block, and agarose was dissolved. All of the heat block and the resin block and the like were put into a thermostatic apparatus under 70°C environment. In the solution for the amplification reaction of a nucleic acid in which the agarose was dissolved under the temperature condition, hollow fibers protruded from the end of a resin block were dipped every 19 pieces, and the gel solution was absorbed from the opposite end of the hollow fiber 12 times repeatedly, whereby to introduce the solution for the amplification reaction of a nucleic acid into the resin block.

The resin block was cooled to room temperature, and the solution for the amplification reaction of a nucleic acid in the inside was gelated.

### Acquisition of base for use in amplification reaction of nucleic acid by slicing

The above-obtained resin block filled with the gel consisting of the solution for the amplification reaction of a nucleic acid was cut to 500 µm of the thickness using a microtome under the conditions where the surface of the resin block and the slice blade were coated with mineral oil (QIAGEN) (specifically under the conditions where the resin block was dipped in the mineral oil), and 15 pieces of bases for use in the amplification reaction of a nucleic acid were obtained in which total 228 holes of 12 (height) x 19 (width) were regularly arranged.

In addition, the same resin block was cut to 500 µm of the thickness using a microtome under the conditions where the mineral oil was not used, and 15 pieces of similar bases for use in the amplification reaction of a nucleic acid were obtained.

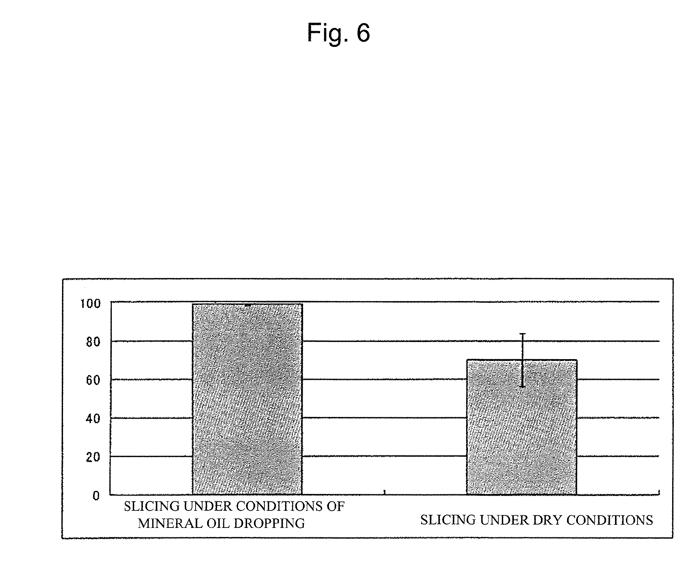

The base for use in the amplification reaction of a nucleic acid obtained by the slicing was observed with an optical microscope, respectively, and the ratio of the agarose gel supported on the hollow fiber in the resin block (the residual ratio) was investigated. The results are shown in Fig. 6.

Specifically, it was shown that a base for use in the amplification reaction of a nucleic acid having stable quality could be obtained by slicing the resin block filled with the gel in a hydrophobic liquid such as mineral oil when agarose gel was used in a spot.

### INDUSTRIAL APPLICABILITY

By using the base and the method of the present invention, it is possible to significantly improve the amplification efficiency or detection efficiency of an organism-related substance that is a subject for detection.

### SEQUENCE TABLE FREE TEXT

SEQ NO: 1: Primer
SEQ NO: 2: Primer
SEQ NO: 3: Primer
SEQ NO: 4: Primer
SEQ NO: 5: Primer
SEQ NO: 6: Primer
SEQ NO: 7: Template

## Claims

1. A base for use in the amplification of a nucleic acid in which a hydrophilic gel that can be dissolved by heating is held, and which has arrayed plural zones,
wherein the zones contain different kinds of primer pairs for amplifying nucleic acids, a DNA polymerase and a buffer component.

2. The base according to claim 1, wherein the concentration of the primer in each of the zones is 1 to 1000 fmol/µL.

3. The base according to claim 1 or 2, wherein the buffer component contains at least one kind selected from the group consisting of KCl, Tris-HCl, MgCl₂, gelatin and Triton X-100.

4. The base according to any one of claims 1 to 3, wherein the hydrophilic gel is a gel prepared using at least one kind selected from the group consisting of agarose, alginic acid, dextran, vinyl alcohol and ethylene glycol as a monomer component.

5. The base according to any one of claims 1 to 3, wherein the hydrophilic gel is an agarose gel.

6. The base according to any one of claims 1 to 5, wherein the concentration of the hydrophilic gel is 0.5 to 2 mass%.

7. The base according to any one of claims 1 to 6, wherein the size of each of the zones is set such that the maximum width of the shape of the zone is 10 µm to 1000 µm.

8. A method for producing the base for use in the amplification of a nucleic acid according to claim 1, which comprises:
(1) a step wherein a plurality of hollow fibers are three-dimensionally arranged so that the fiber axial directions of the hollow fibers become the same, and wherein the arrangement is fixed with a resin to produce a hollow fiber bundle;
(2) a step wherein a plurality of types of gel precursor solutions containing the primers are introduced into the respective hollow portions of the hollow fibers of the hollow fiber bundle that has been heated in advance;
(3) a step wherein the gel precursor solutions introduced into the hollow portions are reacted to hold a gel-like product comprising the primers in the hollow portions of the hollow fibers; and
(4) a step wherein the hollow fiber bundle is sliced in the direction crossing the longitudinal direction of the fibers into thin sections in a hydrophobic liquid.

9. A kit for use in the amplification of a nucleic acid, which comprises the base according to any one of claims 1 to 7, and a base for use in template supply.

10. A method for amplifying a nucleic acid, which comprises a process of supplying a template to each of the zones of the base according to any one of claims 1 to 7, and a process of heating the base after the supply.
